# Europäisches Patentamt
## European Patent Office
## Office européen des brevets

(11) Publication number: **0 022 757**
**B1**

(12) **EUROPEAN PATENT SPECIFICATION**

(45) Date of publication of patent specification: **19.09.84**

(51) Int. Cl.³: **C 12 Q 1/50, C 12 Q 1/66**

(21) Application number: **80850094.6**

(22) Date of filing: **17.06.80**

(54) Method for determining creatine kinase in samples containing ATP

(30) Priority: **12.07.79 SE 7906066**

(43) Date of publication of application:
**21.01.81 Bulletin 81/03**

(45) Publication of the grant of the patent:
**19.09.84 Bulletin 84/38**

(84) Designated Contracting States:
**BE DE FR GB NL**

(56) References cited:
**GB-A-1 163 409**
**GB-A-2 022 247**
**GB-A-2 026 156**
**US-A-3 423 290**
**US-A-4 001 088**
**US-A-4 080 265**

**H.U. BERGMEYER: "Methoden der
enzymatischen Analyse", vol. 2, pages 2163-
2177 Verlag Chemie, 1977, Weinheim, DE B.L.
STREHLER "Adenosin-5'-Triphosphat und
Creatinphosphat. Bestimmung mit Luciferase"**

(73) Proprietor: **LKB-Produkter AB
Box 305
S-161 26 Bromma (SE)**

(72) Inventor: **Lundin, Arne Thorwald
Gaveliusgatan 6
S-116 41 Stockholm (SE)**
Inventor: **Scheuerbrandt, Günter
Im Talgrund 2
D-7821 Breitnau (DE)**

(56) References cited:
**CHEMICAL ABSTRACTS, vol. 92, no. 15, April
14, 1980, page 257, abstract 123845m
Columbus, Ohio, USA LUNDIN et al. "Substrate
and enzyme determinations by continuously
monitoring the ATP level by a purified
luciferase reagent"**
**CHEMICAL ABSTRACTS, vol. 90, no. 5, January
29, 1979, page 221, abstract 35864v, Columbus,
Ohio. USA JABS CLARENCE M. "Plasma ADP
levels: direct determination with luciferase
luminescence using a biometer"**

Courier Press, Leamington Spa, England.

## Description

The present invention refers to a method for determining of the creatine kinase activity in samples containing ATP, for instance blood or other samples containing cells. The invention more specifically refers to the technique in which the enzyme creatine kinase and its substrates ADP and creatine phosphate are brought in contact with a bioluminescence reagent based on firefly luciferase and D-luciferin and the synthesis of ATP is monitored by measuring the light emission generated. The following reaction formulas will apply:

$$ADP + creatine\ phosphate \xrightarrow{\quad creatine\ kinase \quad} ATP + creatine$$

$$ATP + D\text{-luciferin} + O_2 \xrightarrow{\quad firefly\text{-}luciferase \quad} AMP + pyrophosphate + oxiluciferin + CO_2 + light$$

Determining of creatine kinase activity by using the bioluminescence method has been described by Lundin and Styrelius (Clin. Chim. Acta 87 (1978) 199), Lundin, Lindberg, Nordlander, Nyquist and Styrelius (publication presented at the International Symposium on Analytical Applications of Bioluminescence and Chemiluminescence, Brussels, September 1978) and Lundin (Methods in Enzymology (M. DeLuca, ed.) Vol. 57, pages 56—65, Academic Press 1978). Suitable bioluminescence reagents with a stable light emission proportional to the ATP concentration have been described by Lundin and Myhrman (Swedish Patent Application No. 7806296-5). The bioluminescence method for determining creatine kinase activity has through its higher sensitivity, its lower affection of the analysis by turbidity in the samples and the smaller number of coupled reactions considerable advantages as compared to the spectrophotometric routine method for which several kits are commercially available. These features make the bioluminescence method especially suitable for determining creatine kinase activity in samples which have a high background absorption or turbidity at the wave-length used in the spectrophotometric method (340 nm). This is for instance the case in blood samples or other samples containing cells.

The bioluminescence method has previously been used for determining creatine kinase in for instance blood which has been dried in porous vehicles (U.S. Patents Nos. 4,080,265 and 4,001,088). Drying of blood samples on porous vehicles, for instance papers as a method of stabilizing the enzyme activity during sample transportation has been described by Levin and Berezov (Lab. Delo (Moscow) 1972 (3) pages 145—148). In accordance with the above cited bioluminescence method (U.S. Patents Nos. 4,080,265 and 4,001,088) small pieces of paper in which a blood sample has been dried are punched and incubated with a partially purified bioluminescence reagent for 20 hours at 20°C. Thereby, a considerable part of the ATP which is released from the corpuscles is decomposed. This reduces the interference in the analysis from the background of ATP. Finally, one of the substrates in the creatine kinase reaction, namely creatine phosphate is added, and the increase of light emission is measured after 30 minutes. The other substrate, ADP, is not added according to this method. Presumably, the quantities of ADP which have been generated spontaneously during the incubation arysuked. The concentration of ADP during the analysis will in such a case be dependent upon the concentration of the enzyme system which transform adenin nucleotides and which are present in the sample and the reagent. These concentrations can vary in different samples and in different reagent preparations (the bioluminescence reagent according to this method is only partly purified). This may give rise to a variation of the concentration of ADP and thus the creatine kinase activity which gives an uncertainty in the determination of this activity.

According to the present invention the sample containing ATP, for instance blood dried on a porous vehicle is incubated in a buffer solution containing bioluminescence reagent and AMP at a concentration of $10^{-6}$—$10^{-1}$ M, preferably $10^{-4}$—$10^{-3}$ M. This gives rise to a fast decomposition of ATP (Fig. 1) down to a concentration whic does not affect the analysis. Without limiting the invention to any specific reaction mechanics it is possible that the decomposition depends on the adenylate kinase reaction (ATP + AMP → 2 ADP). When the major part of ATP has been decomposed and creatine kinase has been released from a possible porous vehicle (which requires incubation at 25°C for about 15 minutes) both substrates ADP and creatine phosphate added. In order to obtain a good inhibition of the adenylate kinase activity the ADP solution should contain an efficient inhibitor of this enzyme, for instance diadenosin penta phosphate (figure 2). The forming of ATP in adenylate kinase- and creatine kinase reactions is monitored from the light emission. By adding a known quantity of ATP the increase of the light emission could be converted to the forming of ATP per minute, i.e. the enzyme activity.

According to one embodiment of the invention a buffer solution containing A,P and a bioluminescence reagent is added to the sample, this adding being followed by an incubation and further addition of ADP and creatine phosphate. The bioluminescence reagent could however be added after the incubation provided that the buffer solution contains AMP and magnesium ions or other divalent ions which could operate as co-factors in the adenylate kinase reaction without interfering with

2

the later stages of the analysis. It is also possible to add ADP to measure the adenylate kinase activity and thereafter creatine phosphate in order to measure the sum of adenylate kinase- and the creatine kinase activities. This procedure is necessary in situations where the adenylate kinase activity cannot be neglected. When the adenylate kinase activity can be neglected, ADP and creatine phosphate can be added simultaneously which simplifies the analysis. The adenylate kinase inhibitor can then suitably be added in the same reaction stage. The invention also comprises other possible sequences for adding the components necessary for the analysis provided that the addition of AMP increases the decomposition of the ATP background in the samples.

The method according to the invention has the following advantages as compared to the method described in the U.S. patents Nos. 4,080,265 and 4,001,088:

1. The incubation time for decomposing the ATP is reduced from about 20 hours to about 15 minutes.
2. The addition of AMP and an efficient adenylate kinase inhibitor, for instance diadenosin penta phosphate generates an almost complete inhibition of the formation of ATP in the adenylate kinase reaction (2 ADP→ATP+AMP) (compare figure 2).
3. The addition of predetermined quantities of ADP increases the possibilities of obtaining a good reproducability and accuracy within a series of samples and also in a long time perspective.

If a bioluminescence reagent with a stable light emission, for instance in accordance with the Swedish Patent Application 7806296-5 additional advantages appear. If the analysis is performed according to Lundin and Styrelius (Clin. Chim. Acta 87 (1978) 199) the creatine kinase activities as low as 1 U/1 can be determined provided that the adenylate kinase activity does not limit the sensitivity. According to the invention the AMP contributes to the inhibition of the ATP formation in the adenylate kinase reaction. A high sensitivity could be used either for determining low creatine kinase activities or for using small sample volumes.

The most important advantage of a bioluminescence reagent having a stable light emission is that the determination of the creatine kinase activity can be performed kinetically which increases the accuracy. At the creatine kinase activities present for instance in human blood a kinetic analysis could be designed so that the measuring time is below one minute which is an advantage especially if automatic analysis instruments shall be used.

The method according to the invention can be used for determining the creatine kinase activity for instance in blood or other body fluids where an analysis of cells results in an ATP concentration which could interfere with the determination. The technique is not limited to human samples. The samples could either be analysed directly or be dried on porous vehicles. The latter method facilitates the transportation of samples to central laboratories, for instance for screening investigations.

An example of the use of drying on porous vehicles is screening for different muscular human diseases as for instance the Duchenne muscular dystrophy. This disease is one of the most frequent hereditary diseases and affects only boys who slowly atrophy and die usually before the age of 20 years. An extensive screening for this disease by means of creatine kinase determination with the bioluminescence method is now being performed for instance in West Germany. This screening which is voluntary is intended to be used for giving genetic advice to the families affected.

Other applications of the method according to the invention is the screening for the Porcine stress syndrome, which is a disease affecting pigs and giving rise to a bad quality of the meat and thus generates considerable losses.

The method according to the invention can be used for determining of creatine kinase in capillary blood. As serum does not have to be prepared the method is fast and simple and does not require big quantities of sample which is important for instance for cardial infarction patients and children. It should even be possible to design commercial kits for simple determinations of creatine kinase for use in for instance surgeries.

The method will now be described by the following non-limiting example.

Example

This example illustrates the preparation of a reagent kit, the carrying out of an analysis and results from a series of analyses carried out to check a method for screening for human muscular dystrophy with blood dried on paper. The bioluminescence reagent, ATP monitoring reagent, has been prepared according to the Swedish Patent Application No. 7806296-5 and is commercially available as well as the appertaining ATP-standard. The reagent kit is sufficient for 200 determinations and contains the following bottles (one of each category).

ATP Monitoring Reagent (LKB-Wallac, article No. 1250-121) ATP Standard (LKB-Wallac, article No. 1250-122) ADP reagent (1,0 μmol ADP, 0.01 μmol diadenosin penta phosphate) CP reagent (500 μmol creatine phosphate) CK buffer (1.25 mM AMP, 1.25 mM EDTA, 25 mM N-acetyl cystein and 0.125 M imidazole, pH 6.7 regulated with acetic acid).

All reagents except the CK buffer is present in freeze dried form. Before use the ATP monitoring reagent is dissolved in 40 ml CK buffer (this mixture denoted CK-reagent below), ATP standard dissolved in 10 ml distilled water, ADP reagent in 5 ml distilled water and CP-reagent in 5 ml distilled water. The analysis is carried out as follows:

1. A small disc is punched from the dried blood spot. The size of the disc sould be adapted so as to correspond to one or a few microliters of blood.
2. The disc is incubated in 200 $\mu$l CK reagent for about 15 minutes at 25°C so as to decompose the major part of the ATP which is indicated by measuring the light emission.
3. 25 $\mu$l ADP reagent is added and the adenylate kinase activity is determined by measuring the light emission.
4. 25 $\mu$l CP reagent is added and the sum of the adenylate kinase and creatine kinase activities is determined by measuring the light emission.

For screening it is not necessary to determine the adenylate kinase activity. In this case ADP reagent and CP reagent are mixed and 50 $\mu$l of the mixture is added (stages 3 and 4 are carried out simultaneously).

In order to make it possible to transform the increase of light emission dependent on the formation of ATP in the creatine kinase reaction to the increase of ATP concentration, the determinations are finished by an addition of a known ATP concentration and the stepwise increase of the light emission is measured (the internal standard method). As the analysis is not carried out with a saturating ADP concentration it is however better to calibrate the determination by including in each analysis an analysis of a known creatine kinase standard suitably in the form of a dried blood spot on the same vehicle as is used for the sample with human creatine kinase added. A standard curve with such creatine kinase standard is shown in Figure 3.

Figures 1—3 illustrate the above example, thus:

Fig. 1 shows the decomposition of ATP in the presence of AMP (1.25 mM) in a blood sample dried on paper. After decomposition of ATP ADP (20 $\mu$m) and diadenosinpenta phosphate (DAPP; 0.2 $\mu$M and creatine phosphate (CP; 10 mM) are added.

Fig. 2 shows the inhibition of the adenylate kinase reaction with diadenosinpenta phosphate (DAPP; 0.2 $\mu$M). The conditions for the reaction were the same as in Figure 1, but without the addition of DAPP as shown to the left in the figure; and

Fig. 3 shows the correlation between the method according to the invention and the spectrophotometric method for the creatine kinase determination. Creatine kinase standards of dried blood in which the creatine kinase activity in serum were determined spectrophotometrically were analysed with the method according to the invention (conditions for the reaction were the same as in Figure 1).

## Claims

1. Method for determining of creatine kinase in samples containing ATP whereby the sample is brought in contact with ADP, creatine phosphate and a bioluminescence reagent based on firefly luciferase and D-luciferin, characterized in that the sample before the addition of ADP and creatine phosphate is brought in contact with AMP and magnesium ions or other divalent metal ions suitable as cofactors in the adenylate kinase reaction without interfering with the later stages of the analysis, whereafter ADP and creatine phosphate are added and the thus achieved increase of the light emission is measured as a function of time.

2. Method according to claim 1, characterized in that before measuring of the light intensity an efficient adenylate kinase inhibitor is added.

3. Method according to claim 2, characterized in that the adenylate kinase inhibitor is diadenosinpenta phosphate.

## Patentansprüche

1. Verfahren zur Bestimmung von Kreatinkinase in ATP enthaltended Proben, bei dem die Probe mit ADP, Kreatinphosphat und einem Biolumineszenzreagens auf der Basis von Leuchtkäferluciferase und D-Luciferin in Berührung gebracht wird, dadurch gekennzeichnet, daß vor der Zugabe des ADP und des Kreatinphosphats die Probe mit AMP und Magnesiumionen oder anderen zweiwertigen Metallionen, die, ohne die nachfolgenden Analysestufen zu stören, als Kofaktoren in der Adenylatkinasereaktion geeignet sind, in Berührung gebracht wird, wonach das ADP und das Kreatinphosphat zugegeben werden und der auf diese Weise erhaltene Anstieg der Lichtemission als Funktion der Zeit gemessen wird.

2. Verfahren nach Anspruch 1, dadurch gekennzeichnet, daß vor dem Messen der Lichtintensität ein wirksamer Adenylatkinaseinhibitor zugegeben wird.

3. Verfahren nach Anspruch 2, dadurch gekennzeichnet, daß der Adenylatkinaseinhibitor Diadenosinpentaphosphat ist.

# 0 022 757

**Revendications**

1. Méthode de dosage de la créatine kinase dans des échantillons contenant de l'ATP, dans laquelle l'échantillon est mis en contact avec de l'ADP, du phosphate de créatine et un réactif de bioluminescence à base de luciférase de luciole et de D-luciférine, caractérisée en ce que l'échantillon, avant l'addition d'ADP et de phosphate de créatine, est mis en contact avec de l'AMP et des ions magnésium ou d'autres ions de métaux divalents qui conviennent comme cofacteurs dans la réaction de l'adénylate kinase sans interférer avec les derniers stades de l'analyse, après quoi de l'ADP et du phosphate de créatine sont ajoutés et l'accroissement ainsi obtenu de l'émission de lumière est mesuré en fonction du temps.

2. Méthode suivant la revendication 1, caractérisée en ce qu'un inhibiteur efficace d'adénylate kinase est ajouté avant la mesure de l'intensité lumineuse.

3. Méthode suivant la revendication 2, caractérisée en ce que l'inhibiteur d'adénylate kinase est le pentaphosphate de diadénosine.

5